(19) Europäisches Patentamt
European Patent Office
Office européen des brevets

(11) Veröffentlichungsnummer: **0 164 017 A2**

(12) **EUROPÄISCHE PATENTANMELDUNG**

(21) Anmeldenummer: **85106249.7**

(22) Anmeldetag: **22.05.85**

(51) Int. Cl.[4]: **C 07 D 239/46**, C 07 D 249/14, C 07 D 239/42, C 07 D 413/12, A 01 N 47/36 // C07C143/78, C07F7/08

(30) Priorität: **04.06.84 DE 3420769**

(43) Veröffentlichungstag der Anmeldung: **11.12.85 Patentblatt 85/50**

(84) Benannte Vertragsstaaten: **AT BE CH DE FR GB IT LI NL**

(71) Anmelder: **BAYER AG, Konzernverwaltung RP Patentabteilung, D-5090 Leverkusen 1 Bayerwerk (DE)**

(72) Erfinder: **Stetter, Jörg, Dr., Gellertweg 4, D-5600 Wuppertal 1 (DE)**
Erfinder: **Wroblowsky, Heinz-Jürgen, Dr., Gladbacher Strasse 34, D-4018 Langenfeld (DE)**
Erfinder: **Schmidt, Robert R., Dr., Im Waldwinkel 110, D-5060 Bergisch-Gladbach 2 (DE)**
Erfinder: **Santel, Hans-Joachim, Dr., Gerstenkamp 19, D-5000 Köln 80 (DE)**
Erfinder: **Hänssler, Gerd, Dr., Heymannstrasse 40, D-5090 Leverkusen 1 (DE)**
Erfinder: **Lürssen, Klaus, Dr., August-Kierspel-Strasse 151, D-5060 Bergisch-Gladbach 2 (DE)**

(54) **Substituierte Phenylsulfonylharnstoffe.**

(57) Die Erfindung betrifft neue substituierte Phenylsulfonylharnstoffe der allgemeinen Formel

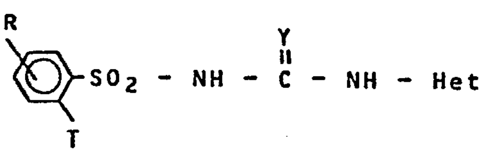 (I)

in welcher
Y für Sauerstoff oder Schwefel steht,
Het für einen heterocyclischen Rest der Formel

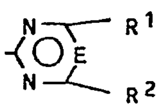 oder 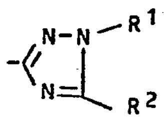 steht,

wobei E für Stickstoff oder die CH-Gruppe steht, und $R^1$ und $R^2$ unabhängig voneinander jeweils für Alkyl, Alkoxy oder Alkoxyalkyl stehen und die Substituenten R und T die in der Beschreibung angegebene Bedeutung haben,

Verfahren zu ihrer Herstellung und ihre Verwendung als Pflanzenbehandlungsmittel, insbesondere als Herbizide, Pflanzenwachstumsregulatoren und Fungizide.

0 164 017



BAYER AKTIENGESELLSCHAFT
Konzernverwaltung RP
Patentabteilung

5090 Leverkusen, Bayerwerk
Bi/Ke-c
Ib

## Substituierte Phenylsulfonylharnstoffe

Die Erfindung betrifft neue substituierte Phenylsulfonylharnstoffe, mehrere Verfahren zu ihrer Herstellung und ihre Verwendung als Pflanzenbehandlungsmittel, insbesondere als Herbizide, Pflanzenwachstumsregulatoren und Fungizide.

Es ist bereits bekannt, daß bestimmte substituierte Phenylsulfonylharnstoffe, wie beispielsweise der N-(2-Cyanmethoxyphenylsulfonyl)-N'-(4,6-dimethylpyrimidin-2-yl)-harnstoff, herbizide und pflanzenwachstumsregulierende Eigenschaften besitzen (vergl. z.B. EP-OS 85 028). Die herbiziden und pflanzenwachstumsregulierenden Eigenschaften dieser vorbekannten substituierten Phenylsulfonylharnstoffe sind jedoch insbesondere bei niedrigen Aufwandmengen und -konzentrationen nicht immer in allen Anwendungsbereichen völlig zufriedenstellend.

Über eine fungizide Wirkung derartiger Sulfonylharnstoffe ist bisher nichts bekannt.

Es wurden neue substituierte Phenylsulfonylharnstoffe der allgemeinen Formel (I),

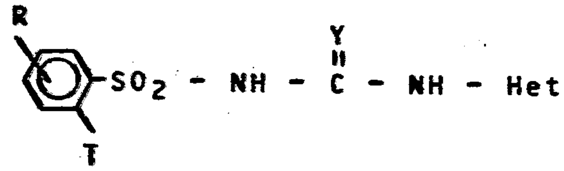 (I)

in welcher

R für Wasserstoff, Halogen, Alkyl, Alkoxy, Halogenalkyl oder Nitro steht,

Y für Sauerstoff oder Schwefel steht,

Het für einen heterocyclischen Rest der Formel

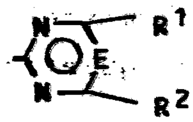 oder 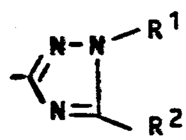 steht,

wobei E für Stickstoff oder die CH-Gruppe steht, und $R^1$ und $R^2$ unabhängig voneinander jeweils für Alkyl, Alkoxy oder Alkoxyalkyl stehen und

T für einen der Reste $-X-CH_2-Si(CH_3)_3$

oder $-X-\overset{X'}{\overset{|}{C}}H-CH=N-OR^3$ steht, wobei

X jeweils für Sauerstoff, Schwefel, die Sulfinylgruppe oder die Sulfonylgruppe steht,

X' für Wasserstoff oder Alkyl steht und

$R^3$ für Wasserstoff, Alkyl, Alkenyl, Alkinyl, Halogenalkyl, Halogenalkenyl, Alkoxyalkyl, Alkylthioalkyl oder für gegebenenfalls substituiertes Aralkyl steht,

und außerdem für einen der Reste

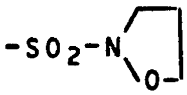 ; 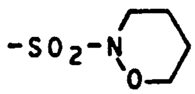 ; $-O-CH_2-CN$ oder

$-O-CH_2-CO-CH_3$ steht, für den Fall, daß Het für einen Heterocyclus der Formel

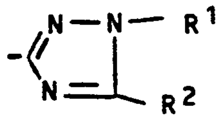 steht, wobei $R^1$ und $R^2$ die oben angegebene Bedeutung haben,

gefunden.

Weiterhin wurde gefunden, daß man die neuen substituierten Phenylsulfonylharnstoffe der Formel (I) erhält, wenn man

(a) heterocyclische Aminoverbindungen der Formel (II)

$$Het - NH_2 \quad (II)$$

in welcher

Het die oben angegebene Bedeutung hat,

mit Phenylsulfonyliso(thio)cyanaten der Formel (III),

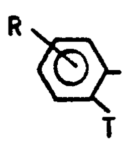 $SO_2 - N = C = Y$ (III)

in welcher

R, Y und T die oben angegebene Bedeutung haben,

gegebenenfalls in Gegenwart eines Verdünnungsmittels und gegebenenfalls in Gegenwart eines basischen Katalysators umsetzt,
oder wenn man

(b) Phenylsulfonamide der Formel (IV)

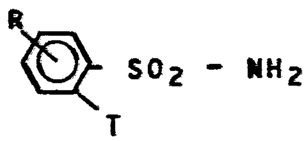 (IV)

in welcher

R und T die oben angegebene Bedeutung haben,

mit (Thio)carbamaten der Formel (V),

$$R^4 - O - \overset{Y}{\overset{\|}{C}} - NH - Het \qquad (V)$$

in welcher

Y und Het die oben angegebene Bedeutung haben und
$R^4$ für Alkyl oder Aryl steht,

gegebenenfalls in Gegenwart eines Verdünnungsmittels und gegebenenfalls in Gegenwart eines Säurebindemittels umsetzt.

Schließlich wurde gefunden, daß die neuen substituierten Phenylsulfonylharnstoffe der Formel (I) herbizide, insbesondere auch selektiv-herbizide, pflanzenwachstumsregulierende und fungizide Eigenschaften besitzen. Sie können daher als Pflanzenbehandlungsmittel eingesetzt werden. Überraschenderweise zeigen die neuen substituierten Phenylsulfonylharnstoffe der Formel (I) neben einer erheblich verbesserten herbiziden Wirkung gegenüber Schadpflanzen auch eine deutlich verbesserte Kulturpflanzenselektivität gegenüber den aus dem Stand der Technik bekannten Verbindungen, wie beispielsweise dem N-(2-Cyanmethoxyphenylsulfonyl)-N'-(4,6-dimethylpyrimidin-2-yl)-harnstoff, welches eine chemisch und wirkungsmäßig naheliegende Verbindung ist, und darüberhinaus zusätzlich wertvolle pflanzenwachstumsregulatorische und fungizide Wirksamkeiten.

Die erfindungsgemäßen substituierten Phenylsulfonylharnstoffe sind durch die Formel (I) allgemein definiert. Bevorzugt sind Verbindungen der Formel (I), bei welchen

R für Wasserstoff, Fluor, Chlor, Brom, Iod, Nitro sowie jeweils geradkettiges oder verzweigtes Alkyl, Halogenalkyl oder Alkoxy mit jeweils bis zu 4 Kohlenstoffatomen und gegebenenfalls bis zu 9 gleichen oder verschiedenen Halogenatomen steht,

Y für Sauerstoff oder Schwefel steht,

Het für einen heterocyclischen Rest der Formel

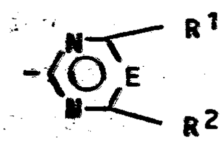 oder 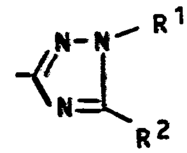 steht, wobei

E für Stickstoff oder die CH-Gruppe steht, und $R^1$ und $R^2$ unabhängig voneinander für jeweils geradkettiges oder verzweigtes Alkyl, Alkoxy oder Alkoxyalkyl mit jeweils bis zu 6 Kohlenstoffatomen in den einzelnen Alkylteilen stehen und

T für einen der Reste $-X-CH_2-Si(CH_3)_3$ oder

$X'$
$-X-CH-CH=N-OR^3$ steht, wobei

X jeweils für Sauerstoff, Schwefel, die Sulfinylgruppe oder die Sulfonylgruppe steht,

X' für Wasserstoff oder für geradkettiges oder verzweigtes Alkyl mit 1 bis 4 Kohlenstoffatomen steht und

$R^3$ für Wasserstoff, für jeweils geradkettiges oder verzweigtes Alkyl, Alkenyl, Alkinyl, Alkoxyalkyl, Alkylthioalkyl, Halogenalkyl oder Halogenalkenyl mit jeweils bis zu 6 Kohlenstoffatomen in den einzelnen Alkyl- bzw. Alkenyl- oder Alkinylteilen und gegebenenfalls bis zu 9 gleichen oder verschiedenen Halogenatomen steht oder für geradkettiges oder verzweigtes, gegebenenfalls im Arylteil einfach oder mehrfach, gleich oder verschieden substituiertes Aralkyl mit bis zu 4 Kohlenstoffatomen im Alkylteil und 6 bis 10 Kohlenstoffatomen im Arylteil steht, wobei als Arylsubstituenten infrage kommen: Halogen, Cyano,

Nitro, sowie jeweils geradkettiges oder verzweigtes Alkyl, Alkoxy oder Halogenalkyl mit jeweils bis zu 4 Kohlenstoffatomen und gegebenenfalls bis zu 9 gleichen oder verschiedenen Halogenatomen,
und außerdem für einen der Reste 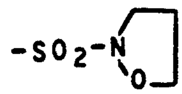 ;

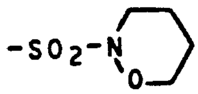 ; $-O-CH_2-CN$ oder $-O-CH_2-CO-CH_3$ steht, -

für den Fall, daß Het für einen Heterocyclus der Formel 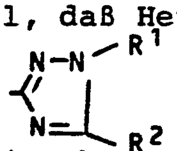 steht, wobei $R^1$ und $R^2$ die oben angegebene Bedeutung haben.

Besonders bevorzugt sind Verbindungen der Formel (I), bei welchen

R für Wasserstoff, Fluor, Chlor, Brom, Nitro, Methyl, Ethyl, n- oder i-Propyl, Trifluormethyl, Methoxy oder Ethoxy steht,

Y für Sauerstoff oder Schwefel steht,

Het für einen heterocyclischen Rest der Formel

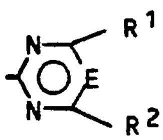 oder 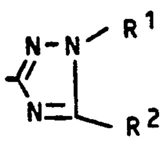 steht, wobei E für Stickstoff oder die CH-Gruppe steht, und $R^1$ und $R^2$ unabhängig voneinander jeweils für Methyl, Ethyl, Methoxy, Ethoxy, Methoxymethyl, Ethoxymethyl, Methoxyethyl oder Ethoxyethyl stehen,

T für einen der Reste $-O-CH_2-Si(CH_3)_3$, $-S-CH_2-Si(CH_3)_3$, $-SO_2-CH_2-Si(CH_3)_3$, $-S-CH_2-CH=N-OR^3$, $-O-\underset{CH_3}{CH}-CH=N-OR^3$, $-S-\overset{CH_3}{CH}-CH=N-OR^3$ oder $-O-CH_2-CH=N-OR^3$ steht, wobei

$R^3$ für Wasserstoff, Methyl, Ethyl, n- oder i-Propyl, n-, i-, s- oder t-Butyl, Allyl, Dichlorallyl, Butenyl, Propargyl, Methoxymethyl, Methoxyethyl, Ethoxyethyl, Methylthiomethyl, Methylthioethyl, Ethylthioethyl oder für jeweils gegebenenfalls ein- bis dreifach, gleich oder verschieden im Phenylteil durch Fluor, Chlor, Brom, Cyano, Nitro, Methyl, Ethyl, Methoxy, Ethoxy oder Trifluormethyl substituiertes Benzyl oder Phenylethyl steht,

und außerdem für einen Rest 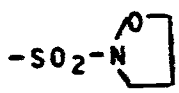 ; 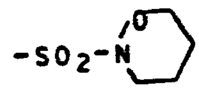 ;

$-O-CH_2-CN$ oder $-O-CH_2-CO-CH_3$ steht, für den Fall daß Het für einen Heterocyclus der Formel 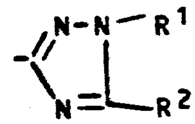 steht,

wobei $R^1$ und $R^2$ die oben angegebene Bedeutung haben.

Im einzelnen seien außer den bei den Herstellungsbeispielen genannten Verbindungen die folgenden substituierten Phenylsulfonylharnstoffe der Formel (I) genannt:

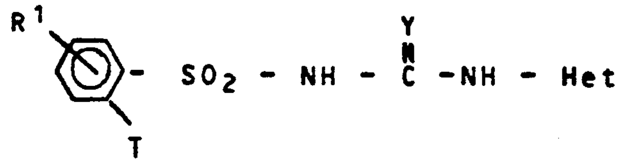 (I)

Het= 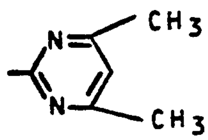 ≡ A ; 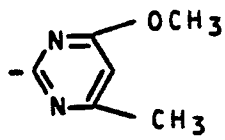 ≡ B ; 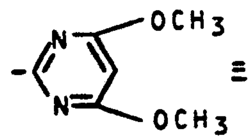 ≡ C

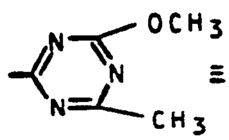 ≡ D ; 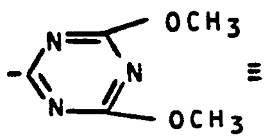 ≡ E; 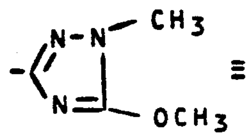 ≡ F

Tabelle 1

| $R^1$ | T | Y | Het |
|---|---|---|---|
| H | $(CH_3)_3Si-CH_2-O-$ | O | A |
| H | $(CH_3)_3Si-CH_2-O-$ | O | C |
| H | $(CH_3)_3Si-CH_2-O-$ | O | D |
| H | $(CH_3)_3Si-CH_2-O-$ | O | E |
| H | $(CH_3)_3Si-CH_2-O-$ | O | A |
| H | $(CH_3)_3Si-CH_2-O-$ | S | B |
| H | $(CH_3)_3Si-CH_2-O-$ | S | C |
| H | $(CH_3)_3Si-CH_2-O-$ | S | E |
| H | $(CH_3)_3Si-CH_2-O-$ | S | D |
| H | $(CH_3)_3Si-CH_2-O-$ | O | F |
| H | $(CH_3)_3Si-CH_2-O-$ | S | F |
| H | $(CH_3)_3Si-CH_2-S-$ | O | A |
| H | $(CH_3)_3Si-CH_2-S-$ | O | B |
| H | $(CH_3)_3Si-CH_2-S-$ | O | C |
| H | $(CH_3)_3Si-CH_2-S$ | O | D |
| H | $(CH_3)_3Si-CH_2-S-$ | O | E |
| H | $(CH_3)_3Si-CH_2-S-$ | O | F |
| H | $(CH_3)_3Si-CH_2-SO_2-$ | O | A |
| H | $(CH_3)_3Si-CH_2-SO_2-$ | O | B |
| H | $(CH_3)_3Si-CH_2-SO_2-$ | O | C |
| H | $(CH_3)_3Si-CH_2-SO_2-$ | O | D |
| H | $(CH_3)_3Si-CH_2-SO_2-$ | O | E |
| H | $(CH_3)_3Si-CH_2-SO_2-$ | O | F |
| H | $CH_3O-N=CH-CH_2-O-$ | O | C |
| H | $CH_3O-N=CH-CH_2-O-$ | O | D |
| H | $CH_3O-N=CH-CH_2-O-$ | O | E |
| H | $CH_3O-N=CH-CH(CH_3)-O-$ | O | A |

Tabelle 1 (Fortsetzung)

| $R^1$ | T | Y | Het |
|---|---|---|---|
| H | $CH_3O-N=CH-CH-O-$<br>$CH_3$ | O | B |
| H | $CH_3O-N=CH-CH-O-$<br>$CH_3$ | O | C |
| H | $CH_3O-N=CH-CH-O-$<br>$CH_3$ | O | D |
| H | $CH_3O-N=CH-CH-O-$<br>$CH_3$ | O | E |
| H | $CH_3O-N=CH-CH-O-$<br>$CH_3$ | O | F |
| H | $CH_3ON=CH-CH_2-S-$ | O | A |
| H | $CH_3ON=CH-CH_2-S-$ | O | B |
| H | $CH_3ON=CH-CH_2-S-$ | O | C |
| H | $CH_3ON=CH-CH_2-S-$ | O | D |
| H | $CH_3ON=CH-CH_2-S-$ | O | E |
| H | $CH_3$<br>$CH_3O-N=CH-CH-S-$ | O | A |
| H | $CH_3$<br>$CH_3O-N=CH-CH-S-$ | O | B |
| H | $CH_3$<br>$CH_3O-N=CH-CH-S-$ | O | C |
| H | $CH_3O-N=CH-CH_2-SO_2-$ | O | A |
| H | $CH_3O-N=CH-CH_2-SO_2-$ | O | B |
| H | $CH_3O-N=CH-CH_2-SO_2-$ | O | C |
| H | $CH_3O-N=CH-CH_2-SO_2-$ | O | D |
| H | $CH_3O-N=CH-CH_2-SO_2-$ | O | E |
| H | $CH_3O-N=CH-CH_2-SO_2-$ | O | F |

Tabelle 1 (Fortsetzung)

| $R^1$ | T | Y | Het |
|---|---|---|---|
| H | $CH_3$<br>\|<br>$CH_3O-N=CH-CH-SO_2-$ | O | A |
| H | $CH_3$<br>\|<br>$CH_3O-N=CH-CH-SO_2-$ | O | B |
| H | $CH_3$<br>\|<br>$CH_3O-N=CH-CH-SO_2-$ | O | C |
| H | $CH_3$<br>\|<br>$CH_3O-N=CH-CH-SO_2-$ | O | D |
| H | $CH_3O-N=CH-CH_2-O-$ | S | A |
| H | $CH_3O-N=CH-CH_2-O-$ | S | B |
| H | $CH_3O-N=CH-CH_2-O-$ | S | C |
| H | $C_2H_5O-N=CH-CH_2-O-$ | O | B |
| H | $C_2H_5O-N=CH-CH_2-O-$ | S | B |
| H | $C_2H_5O-N=CH-CH_2-O-$ | O | A |
| H | $C_2H_5O-N=CH-CH_2-O-$ | O | E |
| H | $CH_3-CO-CH_2-O-$ | S | F |
| H | $NC-CH_2-O-$ | S | F |
| H | 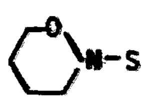 $-SO_2-$ | S | F |
| H | 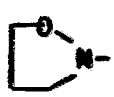 $-SO_2-$ | S | F |
| H | $CH_3(CH_2)_2-O-N=CH-CH_2-O-$ | O | A |
| H | $CH_3(CH_2)_2-O-N=CH-CH_2-O-$ | O | B |
| H | $CH_3(CH_2)_2-O-N=CH-CH_2-O-$ | O | C |
| H | $CH_3(CH_2)_2-O-N=CH-CH_2-O-$ | O | B |
| H | $CH_3(CH_2)_2-O-N=CH-CH_2-O-$ | S | B |
| H | $CH_3(CH_2)_2-O-N=CH-CH_2-O-$ | O | E |

Tabelle 1 (Fortsetzung)

| $R^1$ | T | Y | Het |
|---|---|---|---|
| H | $CH_3(CH_2)_2$-O-N=CH-$CH_2$-O- | S | D |
| H | $CH_3$=CH-$CH_2$-O-N=CH-$CH_2$-O- | O | F |
| H | $CH_2$=CH-$CH_2$-O-N=CH-$CH_2$-O- | S | F |
| H | $CH_2$=CH-$CH_2$-O-N=CH-$CH_2$-O- | O | A |
| H | $CH_2$=CH-$CH_2$-O-N=CH-$CH_2$-O- | O | B |
| H | $CH_2$=CH-$CH_2$-O-N=CH-$CH_2$-O- | O | C |
| H | $CH_2$=CH-$CH_2$-O-N=CH-$CH_2$-O- | O | D |
| H | $CH_2$=CH-$CH_2$-O-N=CH-$CH_2$-O- | O | E |
| H | $CH_2$=CH-$CH_2$-O-N=CH-$CH_2$-O- | O | F |

Verwendet man beispielsweise 2-Cyanmethoxyphenylsulfonylisocyanat und 3-Amino-5-methoxy-1-methyl-1,2,4-triazol als Ausgangsstoffe, so läßt sich der Reaktionsablauf des erfindungsgemäßen Verfahrens (a) durch das folgende Formelschema darstellen:

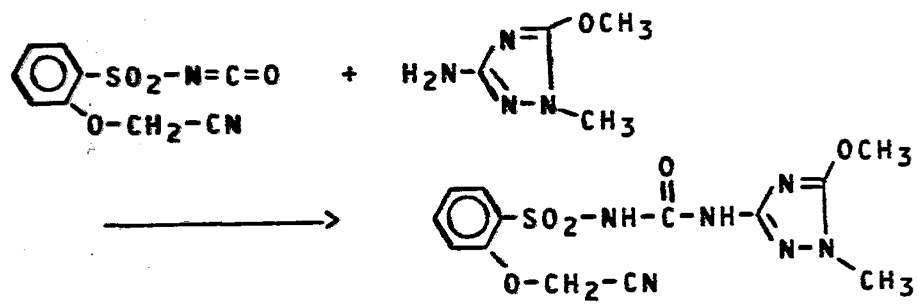

Verwendet man beispielsweise 2-⁅(2-Methoximino)-ethoxy⁆-phenylsulfonamid und N-(4-Methoxy-6-methyl-pyrimidin-2-yl)-O-phenyl-carbamat als Ausgangsstoffe, so läßt sich der Reaktionsablauf des erfindungsgemäßen Verfahrens (b) durch das folgende Formelschema darstellen:

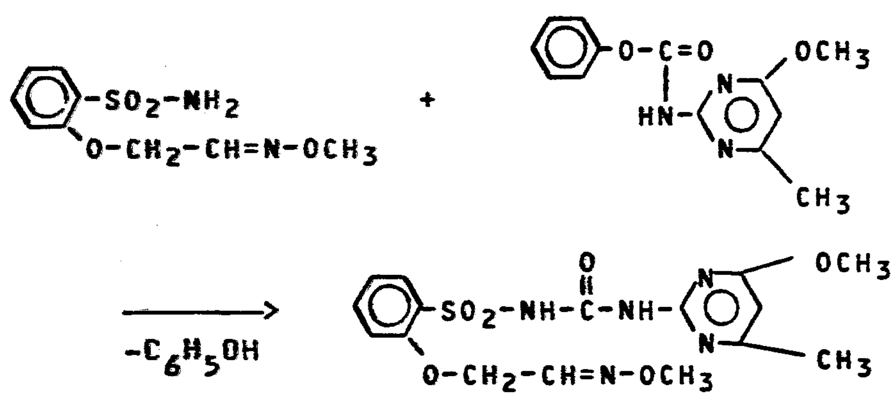

Die als Ausgangsstoffe zur Durchführung des erfindungsgemäßen Verfahrens (a) benötigten heterocyclischen Aminoverbindungen sind durch die Formel (II) allgemein definiert. In dieser Formel (II) steht Het vorzugsweise für diejenigen Reste, die schon bei der Beschreibung der erfindungsgemäßen Stoffe der Formel (I) als bevorzugt für diese Substituenten genannt wurden.

Die heterocyclischen Verbindungen der Formel (II) sind bekannt (vgl. z.B. EP 73 627, EP 73 562, EP 85 028, US-PS 4.127.405).

Die weiterhin zur Durchführung des erfindungsgemäßen Verfahrens (a) als Ausgangsstoffe benötigten Phenylsulfonyliso(thio)cyanate sind durch die Formel (III) allgemein definiert. In dieser Formel (III) stehen R, Y und T vorzugsweise für diejenigen Reste, die schon bei der Beschreibung der erfindungsgemäßen Stoffe der Formel (I) als bevorzugt für diese Substituenten genannt wurden.

Die Phenylsulfonyliso(thio)cyanate der Formel (III) sind teilweise bekannt (vgl. z.B. EP-OS 85 028). Man erhält sie, wenn man substituierte Sulfonamide der Formel (IV),

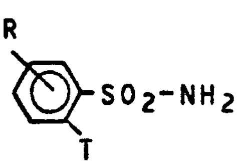

(IV)

in welcher

R und T die oben angegebene Bedeutung haben,

mit Phosgen oder Thiophosgen oder mit Chlorsulfonylisocyanat der Formel (VI),

$$Cl - SO_2 - N = C = O \qquad (VI)$$

gegebenenfalls in Gegenwart eines Verdünnungsmittels, wie beispielsweise Chloroform oder Chlorbenzol, und gegebenenfalls in Gegenwart eines Säurebindemittels, wie beispielsweise Triethylamin, bei Temperaturen zwischen -30°C und +150°C umsetzt.

Die zur Durchführung des erfindungsgemäßen Verfahrens (b) und zur Herstellung der Vorprodukte der Formel (III) als Ausgangsstoffe benötigten Phenylsulfonamide sind durch die Formel (IV) allgemein definiert. In dieser Formel (IV) stehen R und T vorzugsweise für diejenigen Substituenten, die bereits bei der Beschreibung der erfindungsgemäßen Stoffe der Formel (I) als bevorzugt für diese Reste genannt wurden.

Die Phenylsulfonamide der Formel (IV) sind ebenfalls teilweise bekannt (vgl. z.B. EP 85 028). Man erhält sie beispielsweise, wenn man 2-Hydroxy- oder 2-Mercaptophenylsulfonamide der Formel (VII),

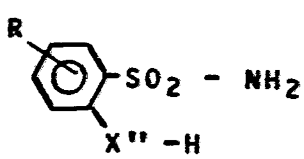

(VII)

in welcher

R die oben angegebene Bedeutung hat und

X" für Sauerstoff oder Schwefel steht,

mit substituierten Halogenalkylverbindungen der Formel (VIII)

$$T' - Hal \qquad (VIII)$$

in welcher

Hal für Halogen, insbesondere Chlor oder Brom steht, und

T' für einen der Reste $-CH_2-Si(CH_3)_3$, $-CH_2-CN$, $-CH_2-CO-CH_3$ oder $-\underset{X'}{\underset{|}{CH}}-CH=N-OR^3$ steht,

wobei $R^3$ und X' vorzugsweise für diejenigen Reste stehen, die schon bei der Beschreibung der erfindungsgemäßen Stoffe der Formel (I) als bevorzugt für diese Substituenten genannt wurden,

gegebenenfalls in Gegenwart eines Verdünnungsmittels, wie beispielsweise Acetonitril, und gegebenenfalls in Gegenwart eines Säurebindemittels, wie beispielsweise Kaliumcarbonat, bei Temperaturen zwischen +20°C und +120°C umsetzt,

und - für den Fall X" = S - die so erhältlichen 2-Mercaptophenylsulfonamide der Formel (IVa)

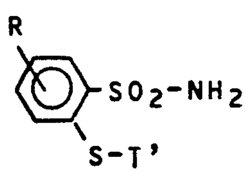

(IVa)

in welcher

R und T' die oben angegebene Bedeutung haben,

gegebenenfalls in einer 2. Stufe mit Oxidationsmitteln, beispielsweise der Formel (IX)

$$H - O - O - R^5 \qquad (IX),$$

in welcher

$R^5$ für Wasserstoff oder einen Acylrest steht, insbesondere für Wasserstoff, Acetyl, Propionyl, Benzoyl, 3-Chlorbenzoyl oder 4-Nitrobenzoyl steht,

gegebenenfalls in Gegenwart eines Verdünnungsmittels, wie beispielsweise Aceton und gegebenenfalls in Gegenwart eines Katalysators wie beispielsweise Ammoniummolybdat bei Temperaturen zwischen -30°C und +50°C in allgemein üblicher Weise oxidiert zu den entsprechenden Sulfinyl- bzw. Sulfonylverbindungen der Formel (IVb),

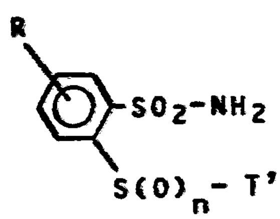

(IVb)

in welcher

T' die oben angegebene Bedeutung hat und

n für 1 oder 2 steht.

Verbindungen der Formel (IVc),

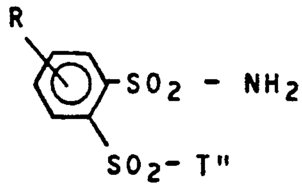

(IVc)

in welcher

R die oben angegebene Bedeutung hat und

T" für einen Heterocyclus der Formel 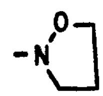 oder 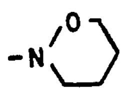 steht,

erhält man, wenn man 2-Chlorsulfonylphenylsulfonsäurefluoride der Formel (X),

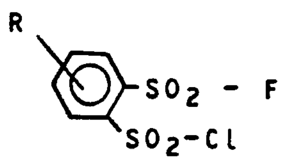

(X)

in welcher

R die oben angegebene Bedeutung hat,

zunächst in einer 1. Stufe mit Heterocyclen der Formel (XI),

H - T" (XI)

in welcher

T" die oben angegebene Bedeutung hat,

gegebenenfalls in Gegenwart eines Verdünnungsmittels wie beispielsweise Toluol und gegebenenfalls in Gegenwart eines Säurebindemittels wie beispielsweise Triethylamin bei Temperaturen zwischen -20°C und +40°C umsetzt, und die so erhaltenen Sulfonylfluoride der Formel (XII),

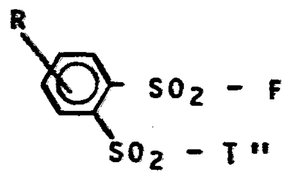

(XII)

in welcher

R und T" die oben angegebene Bedeutung haben.

in einer 2. Stufe mit Ammoniak oder wäßrigem Ammoniumhydroxid bei Temperaturen zwischen +20°C und +60°C umsetzt.

Die zur Durchführung des erfindungsgemäßen Verfahrens (b) weiterhin als Ausgangsstoffe benötigten (Thio)carbamate sind durch die Formel (V) allgemein definiert. In dieser Formel (V) stehen Y und Het vorzugsweise für diejenigen Substituenten, die bereits bei der Beschreibung der erfindungsgemäßen Stoffe der Formel (I) als bevorzugt für diese Reste genannt wurden. $R^4$ steht vorzugsweise für Methyl, Ethyl oder Phenyl.

Die (Thio)carbamate der Formel (V) sind bekannt (vgl. z.B. EP 70 802, EP 70 804, EP 71 958, EP 72 347 oder EP 79 683) oder lassen sich nach bekannten Methoden in einfachen Analogieverfahren herstellen.

Chlorsulfonylisocyanat der Formel (VI), 2-Hydroxyphenylsulfonamide der Formel (VII), substituierte Halogenalkylverbindungen der Formel (VIII), 2-Chlorsulfonylphenylsulfonsäurefluoride der Formel (X) und Heterocyclen der Formel (XI) sind allgemein bekannte Verbindungen der organischen Chemie oder lassen sich nach allgemein bekannten Verfahren in einfacher analoger Weise herstellen.

Als Verdünnungsmittel zur Durchführung der erfindungsgemäßen Verfahren (a) und (b) kommen vorzugsweise inerte organische Lösungsmittel infrage.

Hierzu gehören insbesondere aliphatische oder aromatische, gegebenenfalls halogenierte Kohlenwasserstoffe, wie beispielsweise Benzin, Benzol, Toluol, Xylol, Chlorbenzol, Petrolether, Hexan, Cyclohexan, Dichlormethan, Chloroform, Tetrachlorkohlenstoff, Ether, wie Diethylether, Dioxan, Tetrahydrofuran oder Ethylenglykoldimethyl- oder -diethylether, Ketone wie Aceton oder Butanon, Nitrile, wie Acetonitril oder Propionitril, Amide, wie Dimethylformamid, Dimethylacetamid, N-Methylformanilid, N-Methylpyrrolidon oder Hexamethylphosphorsäuretriamid, Ester, wie Essigsäureethylester oder Sulfoxide, wie Dimethylsulfoxid.

Die erfindungsgemäßen Verfahren (a) und (b) können gegebenenfalls in Gegenwart eines basischen Katalysators durchgeführt werden.

Als solche kommen alle üblichen anorganischen oder organischen Basen infrage. Hierzu gehören beispielsweise Alkalimetallhydroxide, wie Natriumhydroxid oder Kaliumhydroxid, Alkalimetallcarbonate, wie Natriumcarbonat, Kaliumcarbonat oder Natriumhydrogencarbonat, sowie tertiäre Amine, wie Triethylamin, N,N-Dimethylanilin, Pyridin, N,N-Dimethylaminopyridin, Diazabicyclooctan (DABCO), Diazabicyclononen (DBN) oder Diazabicycloundecen (DBU).

Die Reaktionstemperaturen können bei der Durchführung der erfindungsgemäßen Verfahren (a) und (b) in einem größeren Bereich variiert werden. Im allgemeinen arbeitet man bei Temperaturen zwischen -20°C und +120°C, vorzugsweise bei Temperaturen zwischen 0°C und +50°C.

Zur Durchführung des erfindungsgemäßen Verfahrens (a) setzt man pro Mol an heterocyclischer Aminoverbindung der Formel (II) im allgemeinen 1,0 bis 1,5 Mol, vorzugsweise 1,0 bis 1,2 Mol an Phenylsulfonyliso(thio)cyanat der Formel (III) sowie gegebenenfalls 0,01 bis 1,0 Mol, vorzugsweise 0,1 bis 1,0 Mol an basischem Katalysator ein.

Zur Durchführung des erfindungsgemäßen Verfahrens (b) setzt man pro Mol an Phenylsulfonamid der Formel (IV) im allgemeinen 1,0 bis 1,5 Mol, vorzugsweise 1,0 bis 1,2 Mol an (Thio)carbamat der Formel (V) und gegebenenfalls

0,01 bis 1,0 Mol, vorzugsweise 0,1 bis 1,0 Mol an basischem Katalysator ein.

Die Aufarbeitung und Isolierung der Endprodukte der Formel (I) erfolgt bei beiden Herstellungsverfahren (a und b) in üblicher Art und Weise.

Die erfindungsgemäßen Wirkstoffe können als Defoliants, Desiccants, Krautabtötungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewendeten Menge ab.

Die erfindungsgemäßen Wirkstoffe können z.B. bei den folgenden Pflanzen verwendet werden:

Dikotyle Unkräuter der Gattungen: Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea.

Dikotyle Kulturen der Gattungen: Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.

Monokotyle Unkräuter der Gattungen: Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera.

Monokotyle Kulturen der Gattungen: Oryza, Zea, Triticum, Hordeum, Avena, Secale, Sorghum, Panicum, Saccharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. auf Industrie- und Gleisanlagen und auf Wegen und Plätzen mit und ohne Baumbewuchs. Ebenso können die Verbindungen zur Unkrautbekämpfung in Dauerkulturen, z.B. Forst-, Ziergehölz-, Obst-, Wein-, Citrus-, Nuß-, Bananen-, Kaffee-, Tee-, Gummi-, Ölpalm-, Kakao-, Beerenfrucht- und Hopfenanlagen und zur selektiven Unkrautbekämpfung in einjährigen Kulturen eingesetzt werden.

Dabei zeigen die erfindungsgemäßen Wirkstoffe neben einer deutlich verbesserten herbiziden Wirksamkeit gegen wichtige Schadpflanzen gleichzeitig eine erheblich verbesserte Verträglichkeit gegenüber wichtigen Kulturpflanzen und können daher als selektive Unkrautbekämpfungsmittel beispielsweise in Getreide eingesetzt werden.

Die erfindungsgemäßen Wirkstoffe greifen in den Metabolismus der Pflanzen ein und können deshalb auch als Wachstumsregulatoren eingesetzt werden.

Für die Wirkungsweise von Pflanzenwachstumsregulatoren gilt nach der bisherigen Erfahrung, daß ein Wirkstoff auch mehrere verschiedenartige Wirkungen auf Pflanzen ausüben kann. Die Wirkungen der Stoffe hängen im wesentlichen ab von dem Zeitpunkt der Anwendung bezogen auf das Entwicklungsstadium der Pflanze sowie von den auf die Pflanzen oder ihre Umgebung ausgebrachten Wirkstoffmengen und von der Art der Applikation. In jedem Fall sollen Wachstumsregulatoren die Kulturpflanzen in bestimmter gewünschter Weise beeinflussen.

Pflanzenwuchsregulierende Stoffe können zum Beispiel zur Hemmung des vegetativen Wachstums der Pflanzen eingesetzt werden. Eine derartige Wuchshemmung ist unter anderem bei Gräsern von wirtschaftlichem Interesse, denn dadurch kann die Häufigkeit der Grasschnitte in Ziergärten, Park- und Sportanlagen, an Straßenrändern, auf Flughäfen oder in Obstanlagen reduziert werden. Von Bedeutung ist auch die Hemmung des Wuchses von krautigen und holzigen Pflanzen an Straßenrändern und in der Nähe von Pipelines oder Überlandleitungen oder ganz allgemein in Bereichen, in denen ein starker Zuwachs der Pflanzen unerwünscht ist.

Wichtig ist auch die Anwendung von Wachstumsregulatoren zur Hemmung des Längenwachstums von Getreide. Hierdurch

wird die Gefahr des Umknickens ("Lagerns") der Pflanzen vor der Ernte verringert oder vollkommen beseitigt. Außerdem können Wachstumsregulatoren bei Getreide eine Halmverstärkung hervorrufen, die ebenfalls dem Lagern entgegenwirkt. Die Anwendung von Wachstumsregulatoren zur Halmverkürzung und Halmverstärkung erlaubt es, höhere Düngermengen auszubringen, um den Ertrag zu steigern, ohne daß die Gefahr besteht, daß das Getreide lagert.

Eine Hemmung des vegetativen Wachstums ermöglicht bei vielen Kulturpflanzen eine dichtere Anpflanzung, so daß Mehrerträge bezogen auf die Bodenfläche erzielt werden können. Ein Vorteil der so erzielten kleineren Pflanzen ist auch, daß die Kultur leichter bearbeitet und beerntet werden kann.

Eine Hemmung des vegetativen Wachstums der Pflanzen kann auch dadurch zu Ertragssteigerungen führen, daß die Nährstoffe und Assimilate in stärkerem Maße der Blüten- und Fruchtbildung zugute kommen als den vegetativen Pflanzenteilen.

Mit Wachstumsregulatoren läßt sich häufig auch eine Förderung des vegetativen Wachstums erzielen. Dies ist von großem Nutzen, wenn die vegetativen Pflanzenteile geerntet werden. Eine Förderung des vegetativen Wachstums kann aber auch gleichzeitig zu einer Förderung des generativen Wachstums führen, dadurch daß mehr Assimilate gebildet werden, so daß mehr oder größere Früchte entstehen.

Ertragssteigerungen können in manchen Fällen durch einen Eingriff in den pflanzlichen Stoffwechsel erreicht werden, ohne daß sich Änderungen des vegetativen Wachstums bemerkbar machen. Ferner kann mit Wachstumsregulatoren eine Veränderung der Zusammensetzung der Pflanzen erreicht werden, was wiederum zu einer Qualitätsverbesserung der Ernteprodukte führen kann. So ist es beispielsweise möglich, den Gehalt an Zucker in Zuckerrüben, Zuckerrohr, Ananas sowie in Zitrusfrüchten zu erhöhen oder den Proteingehalt in Soja oder Getreide zu steigern. Auch ist es beispielsweise möglich, den Abbau erwünschter Inhaltsstoffe, wie z.B. Zucker in Zuckerrüben oder Zuckerrohr, mit Wachstumsregulatoren vor oder nach der Ernte zu hemmen. Außerdem läßt sich die Produktion oder der Abfluß von sekundären Pflanzeninhaltsstoffen positiv beeinflussen. Als Beispiel sei die Stimulierung des Latexflusses bei Gummibäumen genannt.

Unter dem Einfluß von Wachstumsregulatoren kann es zur Ausbildung parthenokarper Früchte kommen. Ferner kann das Geschlecht der Blüten beeinflußt werden. Auch kann eine Sterilität des Pollens erzeugt werden, was bei der Züchtung und Herstellung von Hybridsaatgut eine große Bedeutung hat.

Durch den Einsatz von Wachstumsregulatoren läßt sich die Verzweigung der Pflanzen steuern. Einerseits kann durch Brechen der Apikaldominanz die Entwicklung von Seitentrieben gefördert werden, was besonders im Zierpflanzenbau auch in Verbindung mit einer Wuchshemmung

sehr erwünscht sein kann. Andererseits ist es aber auch möglich, das Wachstum der Seitentriebe zu hemmen. Für diese Wirkung besteht z.B. großes Interesse im Tabakanbau oder bei der Anpflanzung von Tomaten.

Unter dem Einfluß von Wachstumsregulatoren kann der Blattbestand der Pflanzen so gesteuert werden, daß ein Entblättern der Pflanzen zu einem gewünschten Zeitpunkt erreicht wird. Eine derartige Entlaubung spielt bei der mechanischen Beerntung der Baumwolle eine große Rolle, ist aber auch in anderen Kulturen wie z.B. im Weinbau zur Erleichterung der Ernte von Interesse. Eine Entlaubung der Pflanzen kann auch vorgenommen werden, um die Transpiration der Pflanzen vor dem Verpflanzen herabzusetzen.

Ebenso läßt sich mit Wachstumsregulatoren der Fruchtfall steuern. Einerseits kann ein vorzeitiger Fruchtfall verhindert werden. Andererseits kann aber auch der Fruchtfall oder sogar das Abfallen der Blüten bis zu einem gewünschten Maße gefördert werden ("Ausdünnung"), um die Alternanz zu brechen. Unter Alternanz versteht man die Eigenart einiger Obstarten, endogen bedingt von Jahr zu Jahr sehr unterschiedliche Erträge zu bringen. Schließlich ist es möglich, mit Wachstumsregulatoren zum Zeitpunkt der Ernte die zum Ablösen der Früchte erforderlichen Kräfte zu reduzieren, um eine mechanische Beerntung zu ermöglichen oder eine manuelle Beerntung zu erleichtern.

0 164 017

Mit Wachstumsregulatoren läßt sich ferner eine Beschleunigung oder auch Verzögerung der Reife des Erntegutes vor oder nach der Ernte erreichen. Dieses ist von besonderem Vorteil, weil sich dadurch eine optimale Anpassung an die Bedürfnisse des Marktes herbeiführen läßt. Weiterhin können Wachstumsregulatoren in manchen Fällen die Fruchtausfärbung verbessern. Darüber hinaus kann mit Wachstumsregulatoren auch eine zeitliche Konzentrierung der Reife erzielt werden. Damit werden die Voraussetzungen dafür geschaffen, daß z.B. bei Tabak, Tomaten oder Kaffee eine vollständige mechanische oder manuelle Beerntung in einem Arbeitsgang vorgenommen werden kann.

Durch Anwendung von Wachstumsregulatoren kann ferner die Samen- oder Knospenruhe der Pflanzen beeinflußt werden, so daß die Pflanzen, wie z. B. Ananas oder Zierpflanzen in Gärtnereien, zu einem Zeitpunkt keimen, austreiben oder blühen, an dem sie normalerweise hierzu keine Bereitschaft zeigen. Eine Verzögerung des Austriebes von Knospen oder der Keimung von Samen mit Hilfe von Wachstumsregulatoren kann in frostgefährdeten Gebieten erwünscht sein, um Schädigungen durch Spätfröste zu vermeiden.

Schließlich kann mit Wachstumsregulatoren eine Resistenz der Pflanzen gegen Frost, Trockenheit oder hohen Salzgehalt des Bodens induziert werden. Hierdurch wird die Kultivierung von Pflanzen in Gebieten möglich, die hierzu normalerweise ungeeignet sind.

Zusätzlich weisen die erfindungsgemäßen Wirkstoffe eine starke mikrobizide Wirkung auf und können in entsprechenden Aufwandmengen auch zur Bekämpfung von unerwünschten Mikroorganismen praktisch eingesetzt werden. Die Wirkstoffe sind für den Gebrauch als Pflanzenschutzmittel geeignet.

Fungizide Mittel im Pflanzenschutz werden eingesetzt zur Bekämpfung von Plasmodiophoromycetes, Oomycetes, Chytridiomycetes, Zygomycetes, Ascomycetes, Basidiomycetes, Deuteromycetes.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut, und des Bodens.

Die erfindungsgemäßen Wirkstoffe lassen sich mit besonders gutem Erfolg zur Bekämpfung von Reiskrankheiten wie beispielsweise gegen den Erreger der Reisfleckenkrankheit (Pyricularia oryzae) oder gegen Oomyceten einsetzen. Dabei zeigen die erfindungsgemäßen Wirkstoffe neben einer hervorragenden protektiven Wirksamkeit auch eine sehr gute systemische Wirkung.

Die Wirkstoffe können in die üblichen Formulierungen übergeführt werden, wie Lösungen, Emulsionen, Spritzpulver, Suspensionen, Pulver, Stäubemittel, Schäume, Pasten, lösliche Pulver, Granulate, Aerosole, Suspensions-Emulsionskonzentrate, Saatgutpuder, Wirkstoffimprägnierte Natur- und synthetische Stoffe, Feinstverkapselungen in polymeren Stoffen und in Hüllmassen für Saatgut, ferner in Formulierungen mit Brennsätzen, wie Räucherpatronen, -dosen, -spiralen u.ä. sowie ULV-Kalt- und -Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen infrage: Aromaten, wie Xylol, Toluol, oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid; als feste Trägerstoffe

kommen infrage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen infrage: z.B. gebrochene und fraktionierte natürliche Gesteine wie Calcit, Marmor, Bims, Sepiolith, Dolomit sowie synthetische Granulate aus organischen und anorganischen Mehlen sowie Granulate aus organischem Material, wie Sägemehl, Kokosnußschalen, Maiskolben und Tabakstengeln; als Emulgier- und/oder schaumerzeugende Mittel kommen infrage: z.B. nichtionogene und anionische Emulgatoren, wie Polyoxyethylen-Fettsäureester, Polyoxyethylen-Fettalkohol-ether, z.B. Alkylarylpolyglykolether, Alkylsulfonate, Alkylsulfate, Arylsulfonate sowie Eiweißhydrolysate; als Dispergiermittel kommen infrage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können als solche oder in ihren Formulierungen auch in Mischung mit bekannten Herbiziden zur Unkrautbekämpfung Verwendung finden, wobei Fertigformulierungen oder Tankmischung möglich sind.

Für die Mischungen kommen bekannte Herbizide wie z.B. 1-Amino-6-ethylthio-3-(2,2-dimethylpropyl)-1,3,5-triazin-2,4(1H,3H)-dion oder N-(2-Benzthiazolyl)-N,N'-dimethylharnstoff zur Unkrautbekämpfung in Getreide; 4-Amino-3-methyl-6-phenyl-1,2,4-triazin-5(4H)-on zur Unkrautbekämpfung in Zuckerrüben und 4-Amino-6-(1,1-dimethylethyl)-3-methylthio-1,2,4-triazin-5-(4H)-on zur Unkrautbekämpfung in Sojabohnen, infrage. Auch Mischungen mit N,N-Dimethyl-N'-(3-trifluormethylphenyl)-harnstoff, N,N-Dimethyl-N'-(3-chlor-4-methylphenyl)-harnstoff, N,N-Dimethyl-N'-(4-isopropylphenyl)-harnstoff, 4-Amino-6-t-butyl-3-ethyl-thio-1,2,4-triazin-5(4H)-on, 2,4-Dichlorphenoxyessigsäure, 2,4-Dichlorphenoxypropionsäure, (2-Methyl-4-chlorphenoxy)-essigsäure, (4-Chlor-2-methyl-phenoxy)-propionsäure, 2-[4-(3,5-Dichlorpyrid-2-yloxy)-phenoxy]-propionsäure-(2-benzyloxyethylester), -(trimethylsilylmethylester) oder -(2,2-diethoxy-ethylester) oder andere Diphenylether sind möglich. Einige Mischungen zeigen überraschenderweise auch synergistische Wirkung.

Auch eine Mischung mit anderen bekannten Wirkstoffen, wie Fungiziden, Insektiziden, Akariziden, Nematiziden, Schutzstoffen gegen Vogelfraß, Pflanzennährstoffen und Bodenstrukturverbesserungsmitteln ist möglich.

Bei der Verwendung als Herbizide können die Wirkstoffe als solche, in Form ihrer Formulierungen oder den daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Gießen, Spritzen, Sprühen, Streuen.

Die erfindungsgemäßen Wirkstoffe können sowohl vor als auch nach dem Auflaufen der Pflanzen appliziert werden. Sie können auch vor der Saat in den Boden eingearbeitet werden.

Die angewandte Wirkstoffmenge kann in einem größeren Bereich schwanken. Sie hängt im wesentlichen von der Art des gewünschten Effektes ab. Im allgemeinen liegen die Aufwandmengen zwischen 0,001 und 10 kg Wirkstoff pro Hektar Bodenfläche, vorzugsweise zwischen 0,05 und 5 kg pro ha.

Bei der Verwendung als Pflanzenwachstumsregulatoren können die erfindungsgemäßen Wirkstoffe in den Formulierungen ebenfalls in Mischung mit anderen bekannten Wirkstoffen vorliegen, wie Fungizide, Insektizide, Akarizide und Herbizide, sowie in Mischung mit Düngemitteln und anderen Wachstumsregulatoren.

Die Wirkstoffe können als solche, in Form ihrer Formulierungen oder den daraus bereiteten Anwendungsformen, wie gebrauchsfertige Lösungen, emulgierbare Konzentrate, Emulsionen, Schäume, Suspensionen, Spritzpulver, Pasten, lösliche Pulver, Stäubemittel und Granulate, angewendet werden. Die Anwendung geschieht in üblicher Weise, z.B. durch Gießen, Verspritzen, Versprühen, Verstreuen, Verstäuben, Verschäumen, Bestreichen usw. Es ist ferner möglich, die Wirkstoffe nach dem Ultra-Low-Volume-Verfahren auszubringen oder die Wirkstoffzubereitung oder den Wirkstoff selbst in den Boden zu injizieren. Es kann auch das Saatgut der Pflanzen behandelt werden.

Die Aufwandmengen können bei der Verwendung als Pflanzenwachstumsregulatoren in einem größeren Bereich variiert werden. Im allgemeinen verwendet man pro Hektar Bodenfläche 0,001 bis 10 kg, bevorzugt 0,05 bis 5 kg an Wirkstoff.

Für die Anwendungszeit gilt, daß die Anwendung der Wachstumsregulatoren in einem bevorzugten Zeitraum vorgenommen wird, dessen genaue Abgrenzung sich nach den klimatischen und vegetativen Gegebenheiten richtet.

Bei der Verwendung der erfindungsgemäßen Wirkstoffe als Fungizide können diese in den Formulierungen oder in den verschiedenen Anwendungsformen ebenfalls in Mischung mit anderen bekannten Wirkstoffen vorliegen, wie Fungiziden, Bakteriziden, Insektiziden, Akariziden, Wuchsstoffen, Pflanzennährstoffen und Bodenstrukturverbesserungsmitteln.

Die Wirkstoffe können als solche, in Form ihrer Formulierungen oder der daraus durch weiteres Verdünnen bereiteten Anwendungsformen, wie gebrauchsfertige Lösungen, Emulsionen, Suspensionen, Pulver, Pasten und Granulate angewendet werden. Die Anwendung geschieht in üblicher Weise, z.B. durch Gießen, Tauchen, Spritzen, Sprühen, Vernebeln, Verdampfen, Injizieren, Verschlämmen, Verstreichen, Stäuben, Streuen, Trockenbeizen, Feuchtbeizen, Naßbeizen, Schlämmbeizen oder Inkrustieren.

Bei der Behandlung von Pflanzenteilen können die Wirkstoffkonzentrationen in den Anwendungsformen in einem größeren Bereich variiert werden. Sie liegen im allgemeinen zwischen 1 und 0,0001 Gew.-%, vorzugsweise zwischen 0,5 und 0,001 Gew.-%.

Bei der Saatgutbehandlung werden im allgemeinen Wirkstoffmengen von 0,001 bis 50 g je Kilogramm Saatgut, vorzugsweise 0,01 bis 10 g, benötigt.

Bei Behandlung des Bodens sind Wirkstoffkonzentrationen von 0,00001 bis 0,1 Gew.-%, vorzugsweise von 0,0001 bis 0,02 Gew.-%, am Wirkungsort erforderlich.

Die nachfolgenden Beispiele dienen zur weiteren Erläuterung der Erfindung.

Herstellungsbeispiele:

Beispiel 1

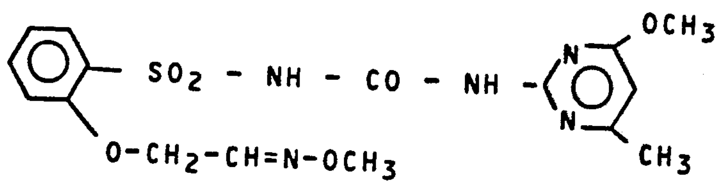

(1)

(Verfahren b)

Zu einer Mischung von 4,9 (0,02 Mol) 2-(2-Methoximino-ethoxy)-phenylsulfonylamid und 5,2 g (0,02 Mol) N-(4-Methoxy-6-methyl-pyrimidin-2-yl)-O-phenyl-carbamat in 160 ml Acetonitril gibt man unter Rühren 3,2 g (0,02 Mol) Diazabicycloundecen (DBU) und rührt weitere 16 Stunden bei Raumtemperatur. Zur Aufarbeitung gießt man den Reaktionsansatz in 600 ml Wasser, säuert mit Salzsäure an, extrahiert mit 500 ml Essigester, wäscht die vereinigten organischen Phasen mit Wasser, trocknet über Natriumsulfat und entfernt das Lösungsmittel im Vakuum. Der ölige Rückstand kristallisiert nach Anreiben mit Essigester. Man erhält 3 g (37 % der Theorie) an N-/2-(2-Methoximinoethoxy)-phenylsulfonyl/-N'-(4-methoxy-6-methyl-pyrimidin-2-yl)-harnstoff vom Schmelzpunkt 191°C.

Herstellung des Vorproduktes

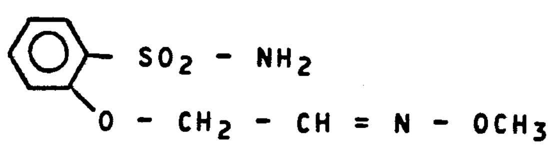

In eine Lösung von 18 g (0,1 Mol) 2-Hydroxybenzolsulfonamid in 20 ml Acetonitril trägt man unter Rühren 30 g (0,2 Mol) Kaliumcarbonat ein und erhitzt anschließend auf 70°C. Dann tropft man schnell 11 g (0,1 Mol) 1-Chlor-2-methoximinoethan zu und erhitzt weitere 3 Stunden auf Siedetemperatur. Der ausgefallene Niederschlag wird abgesaugt, das Filtrat eingedampft, der ölige Rückstand mit Wasser angerieben, abgesaugt und auf Ton getrocknet. Man erhält 14 g (57 % der Theorie) an 2-(2-Methoximinoethoxy)-phenylsulfonamid vom Schmelzpunkt 103 bis 105°C.

Beispiel 2

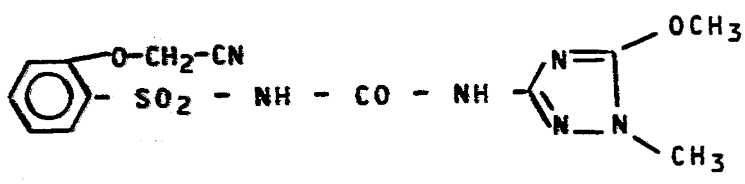

(2)

(Verfahren b)

Zu einer Suspension von 6,4 g (0,03 Mol) 2-Cyanmethoxybenzolsulfonamid und 7,5 g (0,03 Mol) N-(5-Methoxy-1-methyl-1,2,4-triazol-3-yl)-O-phenylcarbamat in 80 ml Acetonitril gibt man unter Rühren 4,8 g (0,03 Mol) Diazabicycloundecen (DBU) und rührt nach beendeter Zugabe weitere 20 Stunden bei Raumtemperatur. Zur Aufarbeitung gießt man den Reaktionsansatz in Wasser, säuert mit Salzsäure an, extrahiert mit 500 ml Essigester, trocknet die vereinigten organischen Phasen über Natriumsulfat und engt im Vakuum ein. Der ölige Rückstand kristallisiert beim Anreiben mit Essigester.

0 164 017

Man erhält 7,8 g (71 % der Theorie) an N-(2-Cyanmethoxyphenylsulfonyl)-N'-(5-methoxy-1-methyl-1,2,4-triazol-3-yl)-harnstoff vom Schmelzpunkt 205 bis 207°C.

Herstellung des Vorproduktes

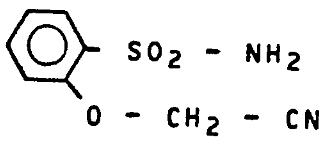

Zu einer Suspension von 18 g (0,1 Mol) 2-Hydroxybenzolsulfonamid und 15 g (0,1 Mol) Kaliumcarbonat in 250 ml Acetonitril tropft man unter Rühren 8 g (0,1 Mol) Chloracetonitril und rührt nach beendeter Zugabe weitere 16 Stunden bei 55°C bis 60°C. Die erhaltene Reaktionsmischung wird abgesaugt, der Rückstand eingedampft und mit Wasser angerieben. Der erhaltene Feststoff wird auf Ton getrocknet. Man erhält 14 g (66 % der Theorie) an 2-Cyanmethoxyphenylsulfonamid vom Schmelzpunkt 184 bis 185°C.

In entsprechender Weise und gemäß den allgemeinen Herstellungsangaben erhält man die folgenden Verbindungen der allgemeinen Formel (I):

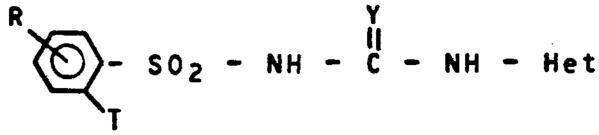

(I)

Tabelle 2:

| Bsp. | R | T | Y | Het | Schmelzpunkt |
|---|---|---|---|---|---|
| 3 | H | $(CH_3)_3SiCH_2-O-$ | O | 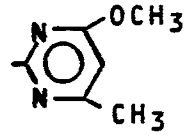 | 100-110°C |
| 4 | H | $CH_3O-N=CHCH_2O-$ | O | 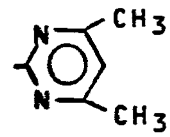 | 196°C |
| 5 | H | 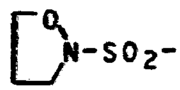 | O | 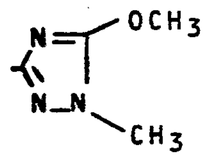 | 202°C |
| 6 | H | 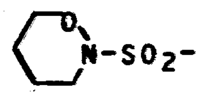 | O | 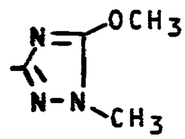 | 197°C |
| 7 | H | $CH_3-CO-CH_2-O-$ | O | 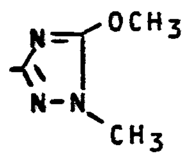 | 172-174°C |

Anwendungsbeispiele:

In den folgenden Anwendungsbeispielen werden die nachstehend aufgeführten Verbindungen als Vergleichssubstanzen eingesetzt:

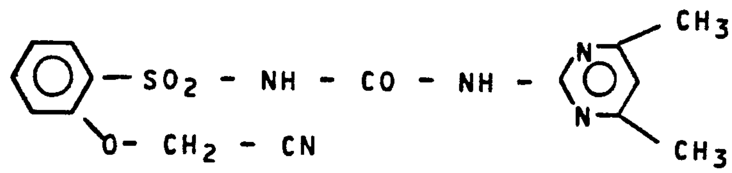

(A)

N-(2-Cyanmethoxyphenylsulfonyl)-N'-(4,6-dimethyl-pyrimidin-2-yl)-harnstoff (bekannt aus EP 85 028)

$$(CH_3-CH_2-CH_2-CH_2-S)_3P = O \qquad (B)$$

S,S,S-Tributyltrithiophosphat (bekannt aus US-PS 3.089.807)

$$Cl-CH_2 - CH_2 - N(CH_3)_3^{\oplus} \quad Cl^{\ominus} \qquad (C)$$

Trimethyl-2-chlorethyl-ammoniumchlorid (bekannt aus DE-AS 12 94 734)

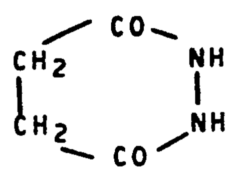

(D)

Maleinsäurehydrazid (bekannt aus Franz.Pat.Nr. 1397521; C.A. 63, 4212a (1965)).

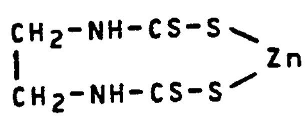

(E)

Zink-ethylen-1,2-bis(dithiocarbamat) (bekannt aus Phytopathology 33, 1113 (1943)).

Beispiel A

Post-emergence-Test

Lösungsmittel: 5 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Mit der Wirkstoffzubereitung spritzt man Testpflanzen, welche eine Höhe von 5 - 15 cm haben so, daß die jeweils gewünschten Wirkstoffmengen pro Flächeneinheit ausgebracht werden. Die Konzentration der Spritzbrühe wird so gewählt, daß in 2000 l Wasser/ha die jeweils gewünschten Wirkstoffmengen ausgebracht werden. Nach drei Wochen wird der Schädigungsgrad der Pflanzen bonitiert in % Schädigung im Vergleich zur Entwicklung der unbehandelten Kontrolle.
Es bedeuten:

0 % = keine Wirkung (wie unbehandelte Kontrolle)
100 % = totale Vernichtung

Eine deutliche Überlegenheit in der Wirksamkeit ebenso wie in der Kulturpflanzenselektivität gegenüber dem Stand der Technik zeigt in diesem Test z.B. die Verbindung gemäß Herstellungsbeispiel 4.

Beispiel B

Entlaubung und Austrocknung der Blätter bei Baumwolle

Lösungsmittel: 30 Gewichtsteile Dimethylformamid
Emulgator: 1 Gewichtsteil Polyoxyethylen-Sorbitan-Monolaurat

Zur Herstellung einer zweckmäßigen Wirkstoffzubereitung vermischt man 1 Gewichtsteil Wirkstoff mit den angegebenen Mengen Lösungsmittel und Emulgator und füllt mit Wasser auf die gewünschte Konzentration auf.

Baumwollpflanzen werden im Gewächshaus bis zur vollen Entfaltung des 5. Folgeblattes angezogen. In diesem Stadium werden die Pflanzen tropfnaß mit den Wirkstoffzubereitungen besprüht. Nach 1 Woche werden der Blattfall und das Austrocknen der Blätter im Vergleich zu den Kontrollpflanzen bonitiert. Es bedeuten:

0 kein Austrocknen der Blätter, kein Blattfall
+ leichtes Austrocknen der Blätter, geringer Blattfall
++ starkes Austrocknen der Blätter, starker Blattfall
+++ sehr starkes Austrocknen der Blätter, sehr starker Blattfall

Eine deutliche Überlegenheit in der Wirksamkeit gegenüber dem Stand der Technik zeigt in diesem Test z.B. die Verbindung gemäß Herstellungsbeispiel 1.

Beispiel C

Wuchshemmung bei Gerste

Lösungsmittel: 30 Gewichtsteile Dimethylformamid
Emulgator: 1 Gewichtsteil Polyoxyethylen-Sorbitan-Monolaurat

Zur Herstellung einer zweckmäßigen Wirkstoffzubereitung vermischt man 1 Gewichtsteil Wirkstoff mit den angegebenen Mengen Lösungsmittel und Emulgator und füllt mit Wasser auf die gewünschte Konzentration auf.

Gerstepflanzen werden im Gewächshaus bis zum 2-Blattstadium angezogen. In diesem Stadium werden die Pflanzen tropfnaß mit den Wirkstoffzubereitungen besprüht. Nach 3 Wochen wird bei allen Pflanzen der Zuwachs gemessen und die Wuchshemmung in Prozent des Zuwachses der Kontrollpflanzen berechnet. Es bedeuten 100 % Wuchshemmung den Stillstand des Wachstums und 0 % ein Wachstum entsprechend dem der Kontrollpflanzen.

Eine deutliche Überlegenheit in der Wirksamkeit gegenüber dem Stand der Technik zeigen in diesem Test z.B. die Verbindungen gemäß folgender Herstellungsbeispiele: 1, 4 und 7.

Beispiel D

Wuchshemmung bei Gras (Festuca pratensis)

Lösungsmittel: 30 Gewichtsteile Dimethylformamid
Emulgator: 1 Gewichtsteil Polyoxyethylen-Sorbitan-Monolaurat

Zur Herstellung einer zweckmäßigen Wirkstoffzubereitung vermischt man 1 Gewichtsteil Wirkstoff mit den angegebenen Mengen Lösungsmittel und Emulgator und füllt mit Wasser auf die gewünschte Konzentration auf.

Gras (Festuca pratensis) wird im Gewächshaus bis zu einer Wuchshöhe von 5 cm angezogen. In diesem Stadium werden die Pflanzen mit den Wirkstoffzubereitungen tropfnaß besprüht. Nach 3 Wochen wird der Zuwachs gemessen und die Wuchshemmung in Prozent des Zuwachses der Kontrollpflanzen berechnet. Es bedeuten 100 % Wuchshemmung den Stillstand des Wachstums und 0 % ein Wachstum entsprechend dem der Kontrollpflanzen.

Eine deutliche Überlegenheit in der Wirksamkeit gegenüber dem Stand der Technik zeigen in diesem Test z.B. die Verbindungen gemäß folgender Herstellungsbeispiele: 1 und 4.

Beispiel E

Pyricularia-Test (Reis) /protektiv

Lösungsmittel: 12,5 Gewichtsteile Aceton
Emulgator: 0,3 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und verdünnt das Konzentrat mit Wasser und der angegebenen Menge Emulgator auf die gewünschte Konzentration.

Zur Prüfung auf protektive Wirksamkeit bespritzt man junge Reispflanzen mit der Wirkstoffzubereitung bis zur Tropfnässe. Nach dem Abtrocknen des Spritzbelages werden die Pflanzen mit einer wäßrigen Sporensuspension von Pyricularia oryzae inokuliert. Anschließend werden die Pflanzen in einem Gewächshaus bei 100 % rel. Luftfeuchtigkeit und 25°C aufgestellt.

4 Tage nach der Inokulation erfolgt die Auswertung des Krankheitsbefalls.

Eine deutliche Überlegenheit in der Wirksamkeit gegenüber dem Stand der Technik zeigen in diesem Test z.B. die Verbindungen gemäß folgender Herstellungsbeispiele: 1, 4 und 7.

Beispiel F

Pyricularia-Test (Reis) /systemisch

Lösungsmittel: 12,5 Gewichtsteile Aceton
Emulgator: 0,3 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und verdünnt das Konzentrat mit Wasser und der angegebenen Menge Emulgator auf die gewünschte Konzentration.

Zur Prüfung auf systemische Eigenschaften werden 40 ml der Wirkstoffzubereitung auf Einheitserde gegossen, in der junge Reispflanzen angezogen wurden. 7 Tage nach der Behandlung werden die Pflanzen mit einer wäßrigen Sporensuspension von Pyricularia oryzae inokuliert. Danach verbleiben die Pflanzen in einem Gewächshaus bei einer Temperatur von 25°C und einer rel. Luftfeuchtigkeit von 100 % bis zur Auswertung.

4 Tage nach der Inokulation erfolgt die Auswertung des Krankheitsbefalls.

Eine deutliche Überlegenheit in der Wirksamkeit gegenüber dem Stand der Technik zeigen in diesem Test z.B. die Verbindungen gemäß folgender Herstellungsbeispiele: 1, 2, 4 und 7.

## Patentansprüche

1. Substituierte Phenylsulfonylharnstoffe der allgemeinen Formel (I)

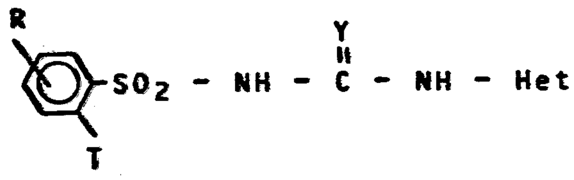

(I)

in welcher

R für Wasserstoff, Halogen, Alkyl, Alkoxy, Halogenalkyl oder Nitro steht,

Y für Sauerstoff oder Schwefel steht,

Het für einen heterocyclischen Rest der Formel

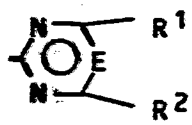 oder 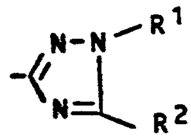 steht,

wobei E für Stickstoff oder die CH-Gruppe steht, und $R^1$ und $R^2$ unabhängig voneinander jeweils für Alkyl, Alkoxy oder Alkoxyalkyl stehen und

T für einen der Reste $-X-CH_2-Si(CH_3)_3$

oder $-X-\overset{X'}{\overset{|}{C}}H-CH=N-OR^3$ steht, wobei

X jeweils für Sauerstoff, Schwefel, die Sulfinylgruppe oder die Sulfonylgruppe steht,

X' für Wasserstoff oder Alkyl steht und

$R^3$ für Wasserstoff, Alkyl, Alkenyl, Alkinyl, Halogenalkyl, Halogenalkenyl, Alkoxyalkyl, Alkylthioalkyl oder für gegebenenfalls substituiertes Aralkyl steht,

und außerdem für einen der Reste

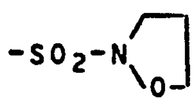 ; 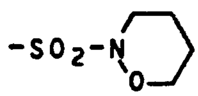 ; $-O-CH_2-CN$ oder

$-O-CH_2-CO-CH_3$ steht, für den Fall, daß Het für einen Heterocyclus der Formel

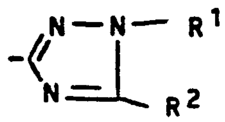 steht, wobei $R^1$ und $R^2$ die oben angegebene Bedeutung haben,

2. Verbindungen der allgemeinen Formel (I) gemäß Anspruch 1, dadurch gekennzeichnet, daß darin

R für Wasserstoff, Fluor, Chlor, Brom, Iod, Nitro sowie jeweils geradkettiges oder verzweigtes Alkyl, Halogenalkyl oder Alkoxy mit jeweils bis zu 4 Kohlenstoffatomen und gegebenenfalls bis zu 9 gleichen oder verschiedenen Halogenatomen steht,

Y für Sauerstoff oder Schwefel steht,

Het für einen heterocyclischen Rest der Formel

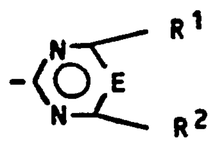 oder 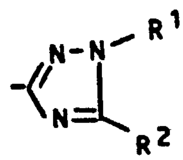 steht, wobei

E für Stickstoff oder die CH-Gruppe steht, und $R^1$ und $R^2$ unabhängig voneinander für jeweils geradkettiges oder verzweigtes Alkyl, Alkoxy oder Alkoxyalkyl mit jeweils bis zu 6 Kohlenstoffatomen in den einzelnen Alkylteilen stehen und

T für einen der Reste $-X-CH_2-Si(CH_3)_3$ oder

$$-X-\underset{}{\overset{X'}{\overset{|}{C}}}H-CH=N-OR^3$$ steht, wobei

X jeweils für Sauerstoff, Schwefel, die Sulfinylgruppe oder die Sulfonylgruppe steht,

X' für Wasserstoff oder für geradkettiges oder verzweigtes Alkyl mit 1 bis 4 Kohlenstoffatomen steht und

$R^3$ für Wasserstoff, für jeweils geradkettiges oder verzweigtes Alkyl, Alkenyl, Alkinyl, Alkoxyalkyl, Alkylthioalkyl, Halogenalkyl oder Halogenalkenyl mit jeweils bis zu 6 Kohlenstoffatomen in den einzelnen Alkyl- bzw. Alkenyl-oder Alkinylteilen und gegebenenfalls bis zu 9 gleichen oder verschiedenen Halogenatomen steht oder für geradkettiges oder verzweigtes, gegebenenfalls im Arylteil einfach oder mehrfach, gleich oder verschieden substituiertes Aralkyl mit bis zu 4 Kohlenstoffatomen im Alkylteil und 6 bis 10 Kohlenstoffatomen im Arylteil steht (wobei als Arylsubstituenten infrage kommen: Halogen, Cyano, Nitro, sowie jeweils geradkettiges oder verzweigtes Alkyl, Alkoxy oder Halogenalkyl mit jeweils bis zu 4 Kohlenstoffatomen und gegebenenfalls bis zu 9 gleichen oder verschiedenen Halogenatomen),

und außerdem für einen der Reste 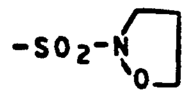 ;

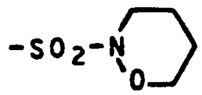 ; $-O-CH_2-CN$ oder $-O-CH_2-CO-CH_3$

steht, -

für den Fall, daß Het für einen Heterocyclus der Formel

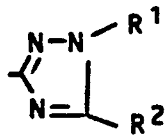

steht, wobei $R^1$ und $R^2$ die oben angegebene Bedeutung haben.

3. Verbindungen der allgmeinen Formel (I) gemäß Anspruch 1, dadurch gekennzeichnet, daß darin

R für Wasserstoff, Fluor, Chlor, Brom, Nitro, Methyl, Ethyl, n- oder i-Propyl, Trifluormethyl, Methoxy oder Ethoxy steht,

Y für Sauerstoff oder Schwefel steht,

Het für einen heterocyclischen Rest der Formel

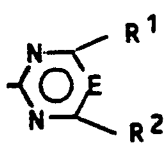 oder 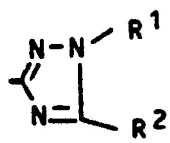 steht, wobei E für Stickstoff oder die CH-Gruppe steht, und $R^1$ und $R^2$ unabhängig voneinander jeweils für Methyl, Ethyl, Methoxy, Ethoxy, Methoxymethyl, Ethoxymethyl, Methoxyethyl oder Ethoxyethyl stehen,

T für einen der Reste $-O-CH_2-Si(CH_3)_3$, $-S-CH_2-Si(CH_3)_3$, $-SO_2-CH_2-Si(CH_3)_3$, $-S-CH_2-CH=N-OR^3$, $-O-CH(CH_3)-CH=N-OR^3$, $-S-CH(CH_3)-CH=N-OR^3$ oder $-O-CH_2-CH=N-OR^3$ steht, wobei

$R^3$ für Wasserstoff, Methyl, Ethyl, n- oder i-Propyl, n-, i-, s- oder t-Butyl, Allyl, Dichlorallyl, Butenyl, Propargyl, Methoxymethyl, Methoxyethyl, Ethoxyethyl, Methylthiomethyl, Methylthioethyl, Ethylthioethyl oder für jeweils gegebenenfalls ein- bis dreifach, gleich oder verschieden im Phenylteil durch Fluor, Chlor, Brom, Cyano, Nitro, Methyl, Ethyl, Methoxy, Ethoxy oder Trifluormethyl substituiertes Benzyl oder Phenylethyl steht,

und außerdem für einen Rest 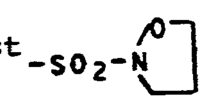 ; 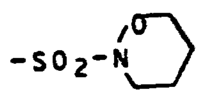 ; $-O-CH_2-CN$ oder $-O-CH_2-CO-CH_3$ steht, für den Fall daß Het für einen Heterocyclus der Formel 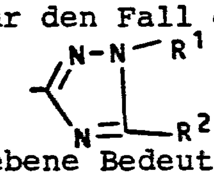 steht, wobei $R^1$ und $R^2$ die oben angegebene Bedeutung haben.

4. Ein substituierter Phenylsulfonylharnstoff, ausgewählt aus der Gruppe, bestehend aus

(a) N-/2-(2-Methoximinoethoxy)-phenylsulfonyl/-N'-(4-methoxy-6-methyl-pyrimidin-2-yl)-harnstoff (1);

(b) N-(2-Cyanmethoxy-phenylsulfonyl)-N'-(5-methoxy-1-methyl-1,2,4-triazol-3-yl)-harnstoff (2);

(c) N-/2̄-(2-Methoximinoethoxy)-phenylsulfonyl1̲7-N'-(4,6-dimethyl-pyrimidin-2-yl)-harnstoff (4) und

(d) N-(2-Acetylmethoxy-phenylsulfonyl)-N'-(5-methoxy-1-methyl-1,2,4-triazin-2-yl)-harnstoff (7)

gemäß Anspruch 1.

5. Verfahren zur Herstellung von substituierten Phenylharnstoffen der allgemeinen Formel (I) gemäß Anspruch 1, dadurch gekennzeichnet, daß man

(a) heterocyclische Aminoverbindungen der Formel (II)

$$Het - NH_2 \qquad (II)$$

in welcher

Het die oben angegebene Bedeutung hat,

mit Phenylsulfonyliso(thio)cyanaten der Formel (III),

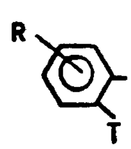 $SO_2 - N = C = Y$ (III)

in welcher

R, Y und T die oben angegebene Bedeutung haben,

gegebenenfalls in Gegenwart eines Verdünnungsmittels und gegebenenfalls in Gegenwart eines basischen Katalysators umsetzt,
oder wenn man

(b) Phenylsulfonamide der Formel (IV)

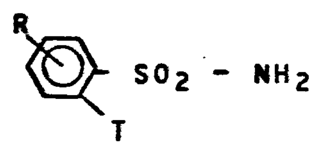

(IV)

in welcher

R und T die oben angegebene Bedeutung haben,

mit (Thio)carbamaten der Formel (V)

$$R^4 - O - \overset{\overset{Y}{\|}}{C} - NH - Het \qquad (V)$$

in welcher

Y und Het die oben angegebene Bedeutung haben und
$R^4$ für Alkyl oder Aryl steht,

gegebenenfalls in Gegenwart eines Verdünnungsmittels und gegebenenfalls in Gegenwart eines Säurebindemittels umsetzt.

6. Pflanzenbehandlungsmittel, gekennzeichnet durch einen Gehalt an mindestens einem substituierten Phenylsulfonylharnstoff der allgemeinen Formel (I) gemäß Anspruch 1.

7. Verwendung von substituierten Phenylsulfonylharnstoffen der allgemeinen Formel (I) gemäß Anspruch 1 als Pflanzenbehandlungsmittel, insbesondere zur Bekämpfung von unerwünschtem Pflanzenwachstum, zur Regulierung des Pflanzenwachstums und zur Bekämpfung von unerwünschten Mikroorganismen, jeweils in geeigneten Konzentrationen bzw. Aufwandmengen.

8. Verfahren zur Herstellung von Pflanzenbehandlungsmitteln, dadurch gekennzeichnet, daß man substituierte Phenylsulfonylharnstoffe der allgemeinen Formel (I) gemäß Anspruch 1 mit Streckmitteln und/ oder oberflächenaktiven Mitteln vermischt.